# EUROPEAN PATENT APPLICATION

(11) **EP 4 693 321 A1**
(43) Date of publication of application: **11.02.2026**
(21) Application number: 24306336.9
(22) Date of filing: 08.08.2024
(51) Int. Cl.: G16H 20/17, G16H 40/63

(54) **METHOD OF PRESSURE MANAGEMENT FOR A DRUG DELIVERY DEVICE**

(71) Applicant: Becton Dickinson France, 38800 Le Pont-de-Claix (FR)
(72) Inventor: MCGRATH, Sean, Artane, Dublin, D05 V5W9 (IE)
(74) Representative: Germain Maureau

(57) **Abstract**

A method of pressure management for a drug delivery device includes delivering fluid through a fluid line via a pump at a first power level, determining a pressure within the fluid line, determining whether the pressure within the fluid line exceeds a pressure threshold level, pausing the delivery of fluid through the fluid line until a predetermined condition is satisfied, and resuming the delivery of the fluid through the fluid line at a second power level after the predetermined condition is satisfied. The second power level is lower than the first power level.

## Description

### BACKGROUND OF THE INVENTION

### Field of the Invention

The present disclosure relates to a method of pressure management for a drug delivery device.

### Description of Related Art

Wearable medical devices, such as automatic injectors, have the benefit of providing therapy to the patient at a location remote from a clinical facility and/or while being worn discretely under the patient's clothing. The wearable medical device can be applied to the patient's skin and configured to automatically deliver a dose of a pharmaceutical composition within a predetermined time period after applying the wearable medical device to the patient's skin, such as after a 27 hour delay. After the device delivers the pharmaceutical composition to the patient, the patient may subsequently remove and dispose of the device.

In certain circumstances, due to the medium the liquid is being injected, the flow of fluid leaving the device may be impaired, which can lead to increased pressure in the fluid line of the device. When the pressure rises above a certain threshold, the integrity of the fluid path may be compromised, causing a leak within the device and a failure to deliver the full dose of medicament. A fluid leak within the device may also cause damage to the device and subsequent system failures, as well as potential contamination concerns due to contact between the fluid and the device.

Human subcutaneous tissue is composed of various cell types, extracellular matrix (ECM) constituents, microstructures, and macroscopic arrangement of cells and ECM. Those elements contribute to the mechanical properties of the tissue. The tissue may also include the lymphatic system, blood vessels, and has intrinsic fluid absorption and retention properties. These characteristics vary among individuals, location within the body, and over time may cause variable degrees of resistance to the infusion of fluids at the site of injection. When the resistance of the tissue is too high or the absorption rate is too low for a given delivery flow rate from the device, the pressure may build up and reach valves above the threshold where the fluid line and other components may be compromised.

### SUMMARY OF THE INVENTION

In one aspect or embodiment, a method of pressure management for a drug delivery device having a pump, a fluid line, and a power source, includes a) delivering fluid through the fluid line via the pump at a first power level; b) determining a pressure within the fluid line; c) determining whether the pressure within the fluid line exceeds a pressure threshold level; d) pausing the delivery of fluid through the fluid line until a predetermined condition is satisfied; e) resuming the delivery of the fluid through the fluid line at a second power level after the predetermined condition is satisfied. The second power level is lower than the first power level.

The predetermined condition may be a predetermined pressure level within the fluid line. The predetermined condition may be a predetermined period of time. The pressure within the fluid line may be determined by measuring a current of the drug delivery device during actuation of the pump.

The measuring of the current of the drug delivery device may include subtracting a reference current value from a peak current value during an actuation cycle of the pump to determine a stroke current value.

The second power level may be provided by modulating the voltage supplied to the pump.

The method may further include f) determining whether the pressure within the fluid line exceeds the pressure threshold level, g) pausing the delivery of fluid through the fluid line until the predetermined condition is satisfied, and h) resuming the delivery of the fluid through the fluid line at a third power level after the predetermined condition is satisfied, with third power level being lower than the second power level.

The first power level may be 100% of full pump current, the second power level may be 75% of full pump current, and the third power level may be 50% of full pump current.

The method may further include selecting one of the first power level, the second power level, the third power level, and the fourth power level based on a number of times delivery of the fluid through the fluid line was previously paused. The third power level is lower than the second power level. The fourth power level is lower than the third power level.

In a further aspect or embodiment, a drug delivery device includes a power source, a reservoir configured to receive a fluid, a fluid line in fluid communication with the reservoir, a pump configured to deliver the fluid from the reservoir to the fluid line, and a microcontroller having at least one processor programmed or configured to cause the device to perform the method according to any of the aspects or embodiments discussed above.

### BRIEF DESCRIPTION OF THE DRAWINGS

The above-mentioned and other features and advantages of this disclosure, and the manner of attaining them, will become more apparent and the disclosure itself will be better understood by reference to the following descriptions of embodiments of the disclosure taken in conjunction with the accompanying drawings, wherein:
**FIG.** 1 is a perspective view of a drug delivery device according to one aspect or embodiment of the present application;
**FIG.** 2 is a perspective view of the drug delivery device of FIG. 1, with a top cover removed;
**FIG.** 3 is a schematic view of the drug delivery device of FIG. 1;
**FIG.** 4 is a schematic view of a method of pressure management according to one aspect or embodiment of the present application;
**FIG.** 5 is a further schematic view of the method of FIG. 5;
**FIG.** 6 is a graph of pressure versus time, showing an exemplary pause in delivery of a fluid after an exemplary pressure threshold has been surpassed; and
**FIG.** 7 is a schematic view of a method of pressure management according to one aspect or embodiment of the present application.

Corresponding reference characters indicate corresponding parts throughout the several views. The exemplifications set out herein illustrate exemplary embodiments of the disclosure, and such exemplifications are not to be construed as limiting the scope of the disclosure in any manner.

### DETAILED DESCRIPTION OF THE INVENTION

Spatial or directional terms, such as "left", "right", "inner", "outer", "above", "below", and the like, are not to be considered as limiting as the invention can assume various alternative orientations.

All numbers used in the specification and claims are to be understood as being modified in all instances by the term "about". By "about" is meant a range of plus or minus ten percent of the stated value. As used in the specification and the claims, the singular form of "a", "an", and "the" include plural referents unless the context clearly dictates otherwise. The terms "first", "second", and the like are not intended to refer to any particular order or chronology, but instead refer to different conditions, properties, or elements. By "at least" is meant "greater than or equal to".

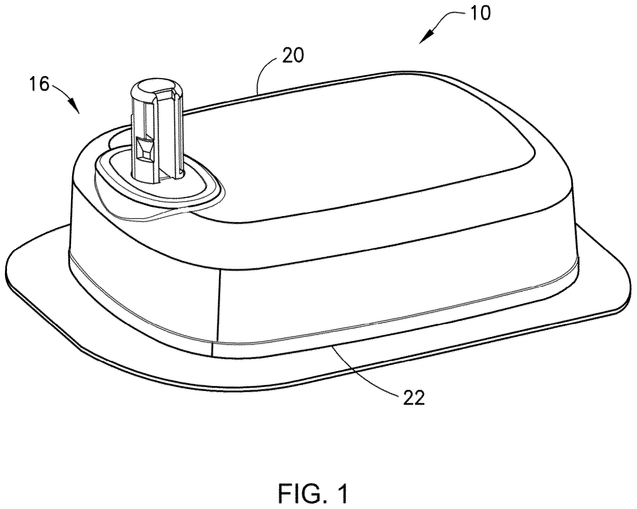

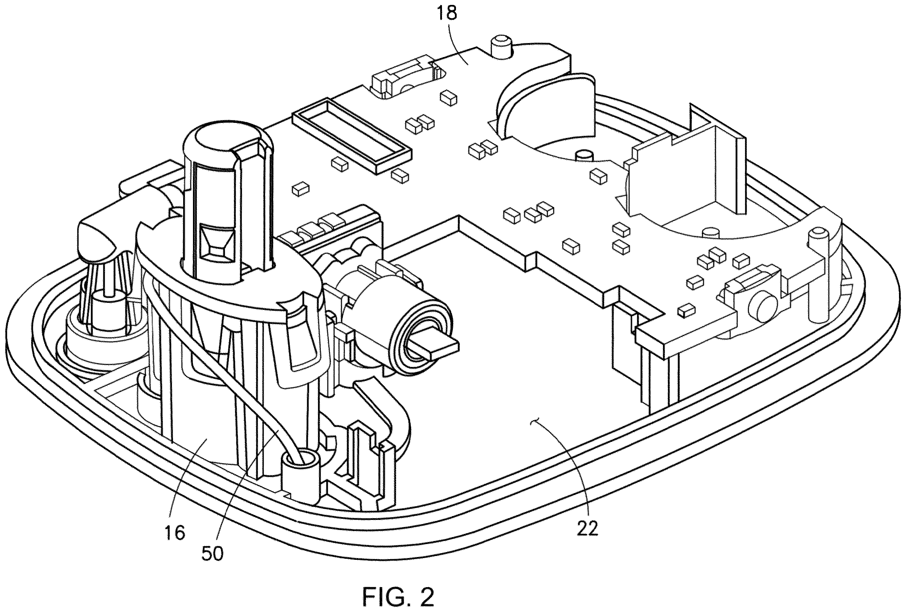

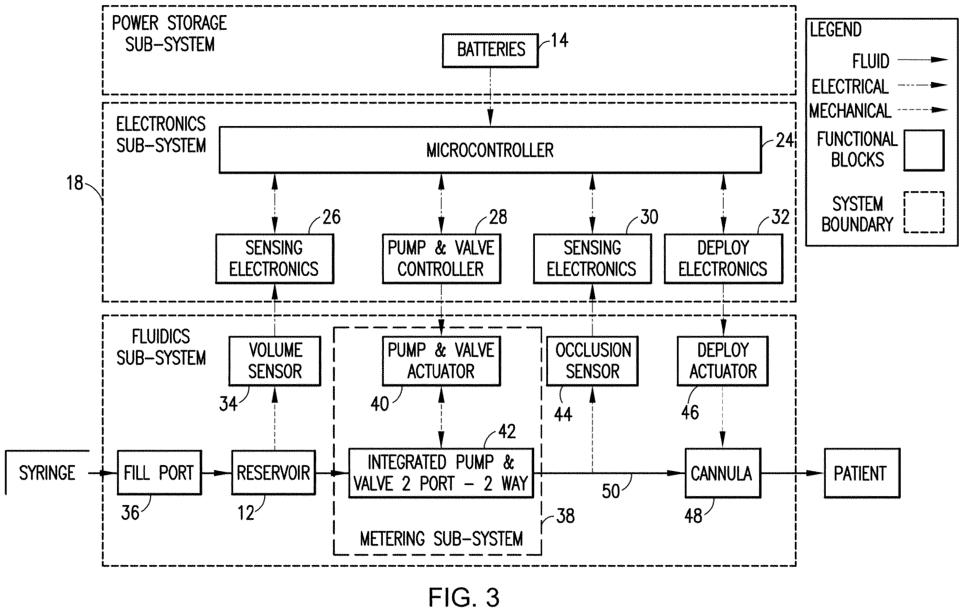

Referring to FIGS. 1-3, a drug delivery device 10 includes a reservoir 12, a power source 14, an insertion mechanism 16, control electronics 18, a cover 20, and a base 22. In one aspect or embodiment, the drug delivery device 10 is a wearable automatic injector, such as an insulin or bone marrow stimulant delivery device. The drug delivery device 10 may be mounted onto the skin of a patient and triggered to inject a pharmaceutical composition from the reservoir 12 into the patient. The drug delivery device 10 may be pre-filled with the pharmaceutical composition, or it may be filled with the pharmaceutical composition by the patient or medical professional prior to use.

The drug delivery device 10 is configured to deliver a dose of a pharmaceutical composition, e.g., any desired medicament, into the patient's body by a subcutaneous injection at a slow, controlled injection rate. Exemplary time durations for the delivery achieved by the drug delivery device 10 may range from about 5 minutes to about 60 minutes, but are not limited to this exemplary range. Exemplary volumes of the pharmaceutical composition delivered by the drug delivery device 10 may range from about 0.1 milliliters to about 10 milliliters, but are not limited to this exemplary range. The volume of the pharmaceutical composition delivered to the patient may be adjusted.

Referring again to FIGS. 1-3, in one aspect or embodiment, the power source 14 is a DC power source including one or more batteries. The control electronics 18 include a microcontroller 24, sensing electronics 26, a pump and valve controller 28, sensing electronics 30, and deployment electronics 32, which control the actuation of the drug delivery device 10. The drug delivery device 10 includes a fluidics sub-system that includes the reservoir 12, volume sensor 34 for the reservoir 12, a reservoir fill port 36, and a metering system 38 including a pump and valve actuator 40 and a pump and valve mechanism 42. The fluidic sub-system may further include an occlusion sensor 44, a deploy actuator 46, a cannula 48 for insertion into a patient's skin, and a fluid line 50 in fluid communication with the reservoir 12 and the cannula 48. In an alternative aspect or embodiment, the occlusion sensor 44 is a system for measuring the motor current. In one aspect or embodiment, the insertion mechanism 16 is configured to move the cannula 48 from a retracted position positioned entirely within the device 10 to an extended position where the cannula 48 extends outside of the device 10. The drug delivery device 10 may operate in the same manner as discussed in U.S. Patent No. 10,449,292 to Pizzochero et al.

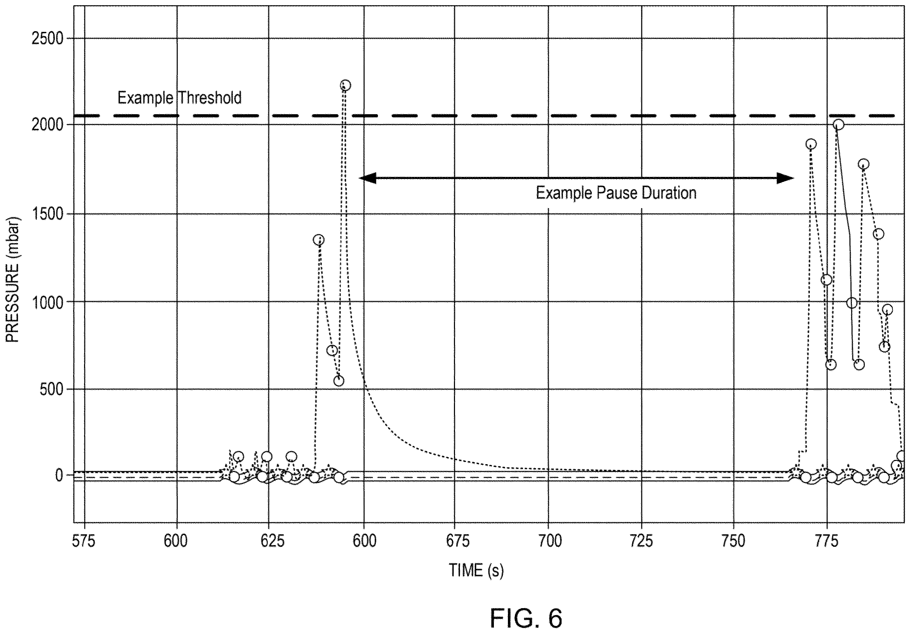

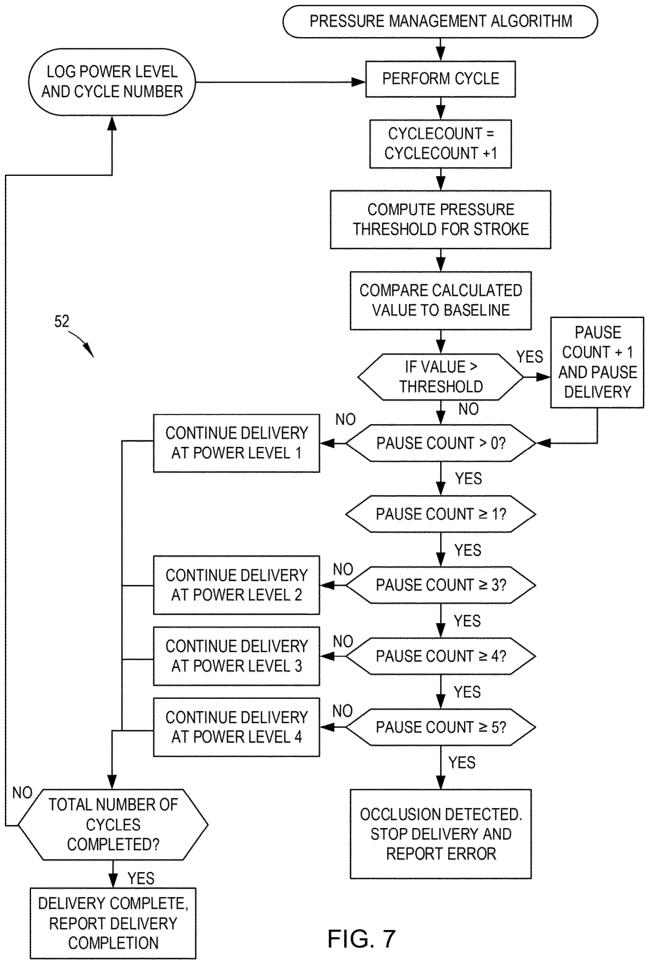

Referring to FIGS. 6 and 7, in one aspect or embodiment, a method 52 of pressure management for the drug delivery device 10 includes: a) delivering fluid through the fluid line 50 via the pump and valve mechanism 42 at a first power level; b) determining a pressure within the fluid line 50; c) determining whether the pressure within the fluid line 50 exceeds a pressure threshold level; d) pausing the delivery of fluid through the fluid line 50 until a predetermined condition is satisfied; e) resuming the delivery of the fluid through the fluid line 50 at a second power level after the predetermined condition is satisfied. The second power level is lower than the first power level. Accordingly, the combination of pausing and power-level adjustment is configured to minimize the total delivery time while also minimizing the incidence of occlusions or high pressure events.

The predetermined condition may be a predetermined period of time, although the predetermined condition may also be a predetermined pressure level within the fluid line 50, or the predetermined condition may be an input from a sensor in the device or an input from a user. The pressure level within the fluid line 50 may be continuously monitored utilizing a sensor or other arrangement, although the pressure may also be determined based on the current utilized during actuation of the device 10, as discussed in additional detail below. As shown in FIG. 6, for example, when the pressure within the fluid line 50 reaches the pressure threshold level, a pause of the delivery of the fluid is initiated to let the pressure naturally decay, when possible, to a level at which the delivery of fluid can be resumed. As shown in Fig. 7, after pausing delivery, delivery of the fluid is resumed at a second power level. The method 52 may include one or more iterative processes from the beginning of the delivery of the fluid to the completion of the delivery of the full dose of fluid. The method 52 may be executed by the microcontroller 24 or other processor of the drug delivery device 10.

In one aspect or embodiment, the method 52 includes terminating the delivery of fluid through the fluid line 50 when the delivery of fluid has been paused for a maximum delay period. For example, if the delivery of the fluid is paused until the pressure within the fluid line 50 falls below the predetermined pressure level and the pressure within the fluid line 50 does not fall below the predetermined pressure level after the maximum delay period, the delivery process is terminated, and an error or occlusion indication may be provided by the drug delivery device 10. In one aspect or embodiment, the maximum delay period is at least 2 minutes. In one aspect or embodiment, the delivery of the fluid is provided via a plurality of smaller deliveries and the maximum delay period includes the total amount of time or total number of delay periods between these discrete deliveries. For example, if the delivery of fluid is paused for the predetermined amount of time due to the pressure within the fluid line 50 exceeding the pressure threshold level, a subsequent bolus may be delivered and, if the pressure continues to exceed the pressure threshold level 60, the delivery may be terminated. The delivery may be terminated after one or more subsequent bolus deliveries when the delivery has been paused and fails to fall below the pressure threshold level.

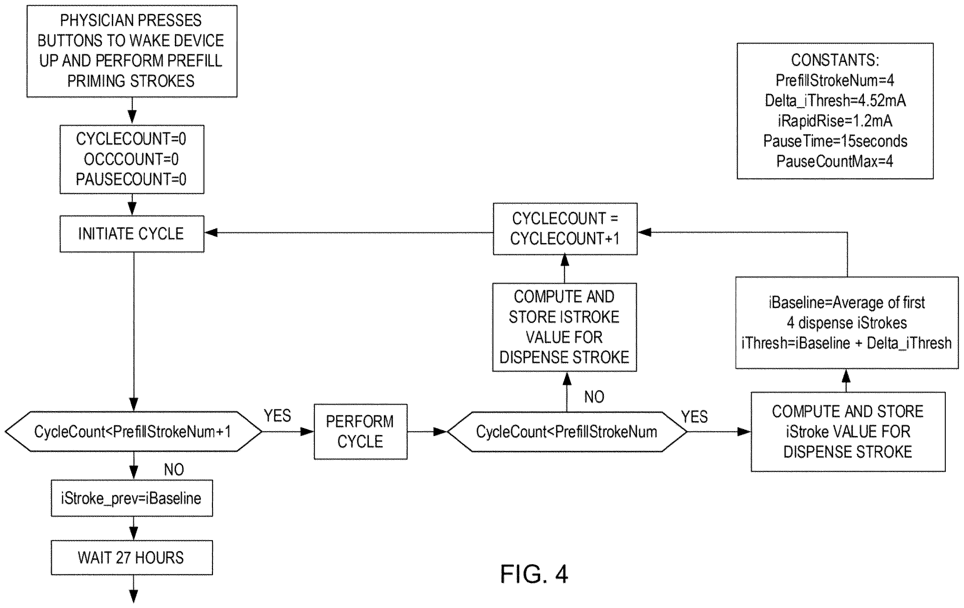

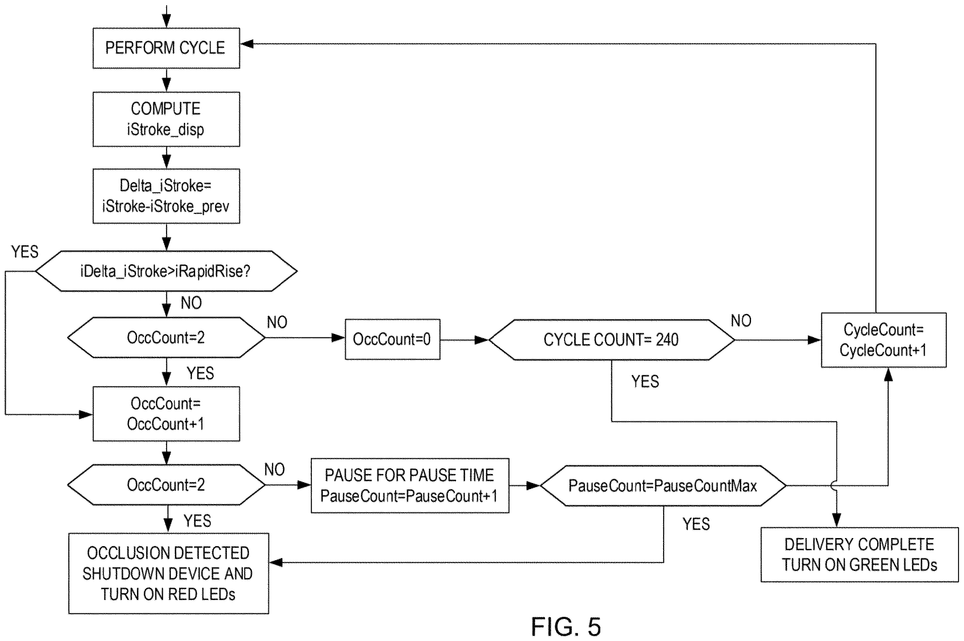

In one aspect or embodiment, the pressure within the fluid line 50 is determined by measuring a current of the drug delivery device 10 during actuation of the pump and valve mechanism 42. The measuring of the current of the drug delivery device 10 includes subtracting a reference or baseline current value from a peak current value during an actuation cycle of the pump and valve mechanism 42 to determine a stroke current value, although other suitable current detection arrangements may be utilized. The stroke current value is utilized to estimate the downstream pressure of the fluid line 50 for the particular actuation cycle of the pump and valve mechanism 42. For example, the stroke current value can correspond to various downstream pressure levels through testing or benchmarking such that the stroke current value can be used to accurately estimate the pressure level of the fluid line 50. In some aspects or embodiments, the determination of the pressure within the fluid line 50 and whether the pressure within the fluid line exceeds a predetermined pressure threshold level can be accomplished using the methodology shown in FIGS. 4 and 5. The methodology shown in FIGS. 4 and 5 shows pausing of deliver and the method 52 according to aspects or embodiments of the present application may utilize similar pause methodology in combination with the reduction of power levels as discussed herein.

A relationship between pressure within the fluid line 50 and current required to push the pump and valve mechanism 42 forward can be determined through testing or benchmarking. In one aspect or embodiment, the pump and valve mechanism 42 has an aspiration cycle and a dispense cycle. In one aspect or embodiment, with a pump current of 1.95 mA, the pressure within the fluid line 50 can be estimated to be approximately 0 psi. In one aspect or embodiment, with a current of 7.61 mA, the pressure within the fluid line 50 can be estimated to be approximately 40 psi. The correlation between the current and the pressure within the fluid line 50 may be determined via testing using a pressure sensor to measure the pressure within the fluid line 50.

In one aspect or embodiment, the drug delivery device 10 includes a power limitation subsystem configured to limit a power level supplied to the pump and valve mechanism 42. The power limitation subsystem may be a current limiter subsystem. The current limiter subsystem is configured to limit or cap the current supplied to the pump and valve mechanism. The current limiter subsystem may utilize PNP transistors and/or NPN transistors. In a further aspect or embodiment, the power limitation subsystem is provided by the microcontroller 24 modulating a voltage supplied to the pump and valve mechanism 42. By using pulse width modulation, the power supplied to the pump and valve mechanism 42 can be modulated. For example, using a narrow pulse over a period of time will result in a lower average voltage than using a wider pulse over a period of time. The pulse width modulated signal may be smoothed by using dedicated circuitry such as capacitors, or may be smoothed by the load formed by the actuator itself.

In one aspect or embodiment, the power limitation subsystem is configured to be adjustable to allow the power level supplied to the pump and valve mechanism 42 to be varied as needed. In one aspect or embodiment, the power limitation subsystem has an activated mode where the power level being supplied is limited and a deactivated mode where the power level being supplied is not limited. The activated mode and the deactivated mode may be provided via additional circuitry and/or by the control via the microcontroller 24.

In one aspect or embodiment, the method 52 may further include: determining whether the pressure within the fluid line 50 exceeds the pressure threshold level; pausing the delivery of fluid through the fluid line 50 until the predetermined condition is satisfied; and resuming the delivery of the fluid through the fluid line 50 at a third power level after the predetermined condition is satisfied. The third power level is lower than the second power level. A fourth power level may be utilized if the pressure within the fluid line 50 continues to exceed the pressure threshold level. In one aspect or embodiment, the first power level may be 100% of full pump current, the second power level may be 75% of full pump current, the third power level may be 50% of full pump current, and the fourth power level may be 25% of full pump current. In one aspect or embodiment, full pump current is a maximum current able to be supplied to the pump and valve mechanism 42 by the batteries 14.

Referring to FIG. 7, in some aspects or embodiments, the method 52 utilizes a condition-controlled or count-controlled loop. The method 52 may utilize a pause count that is iteratively increased if the pressure within the fluid line 50 is found to exceed the pressure threshold level. For example, the pause count has a starting value of zero and is incremented by one every time the pressure within the fluid line 50 is found to exceed the pressure threshold level. As shown in FIG. 7, for example, the delivery of the fluid through the fluid line 50 can resume at the second power level after pausing delivery and if the pause count is two. If the pause count is three, the delivery of the fluid through the fluid line 50 can resume at the third power level after pausing delivery. If the pause count is four, the delivery of the fluid through the fluid line 50 can resume at the fourth power level after pausing delivery. Other suitable pause counts and corresponding power levels may be utilized. As indicated above, if the pause count exceeds a maximum value, such as 5, the delivery of the fluid may be terminated, with an occlusion alarm or indicator provided by the device 10. After each delivery cycle, the power level and the cycle number may be logged. The delivery may be completed after a predetermined number of delivery cycles.

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within the spirit and scope of the appended claims. For example, it is to be understood that the present invention contemplates that, to the extent possible, one or more features of any embodiment can be combined with one or more features of any other embodiment.

## Claims

1. A method of pressure management for a drug delivery device comprising a pump, a fluid line, and a power source, the method comprising:
a) delivering fluid through the fluid line via the pump at a first power level;
b) determining a pressure within the fluid line;
c) determining whether the pressure within the fluid line exceeds a pressure threshold level;
d) pausing the delivery of fluid through the fluid line until a predetermined condition is satisfied; and
e) resuming the delivery of the fluid through the fluid line at a second power level after the predetermined condition is satisfied, wherein the second power level is lower than the first power level.

2. The method of claim 1, wherein the predetermined condition comprises a predetermined pressure level within the fluid line.

3. The method of claim 1, wherein the predetermined condition comprises a predetermined period of time.

4. The method of any of claims 1-3, wherein the pressure within the fluid line is determined by measuring a current of the drug delivery device during actuation of the pump.

5. The method of claim 4, wherein the measuring of the current of the drug delivery device comprises subtracting a reference current value from a peak current value during an actuation cycle of the pump to determine a stroke current value.

6. The method of any of claims 1-5, wherein the second power level is provided by modulating the voltage supplied to the pump.

7. The method of any of claims 1-6, further comprising:
f) determining whether the pressure within the fluid line exceeds the pressure threshold level;
g) pausing the delivery of fluid through the fluid line until the predetermined condition is satisfied; and
h) resuming the delivery of the fluid through the fluid line at a third power level after the predetermined condition is satisfied, wherein the third power level is lower than the second power level.

8. The method of claim 1, wherein the first power level comprises 100% of full pump current and the second power level comprises 75% of full pump current.

9. The method of claim 7, wherein the first power level comprises 100% of full pump current, the second power level comprises 75% of full pump current, and the third power level comprises 50% of full pump current.

10. The method of any of claims 1-9, wherein b) through e) are performed using a microcontroller comprising at least one processor.

11. The method of claim 1, further comprising:
selecting one of the first power level, the second power level, a third power level, and a fourth power level based on a number of times delivery of the fluid through the fluid line was previously paused, wherein the third power level is lower than the second power level, and wherein the fourth power level is lower than the third power level.

12. A drug delivery device comprising:
a power source;
a reservoir configured to receive a fluid;
a fluid line in fluid communication with the reservoir;
a pump configured to deliver the fluid from the reservoir to the fluid line; and
a microcontroller comprising at least one processor programmed or configured to cause the device to perform the method of any of claims 1-11.
